# FASCICULE DE BREVET EUROPEEN

(11) **EP 1 031 830 B1**
(45) Date de publication et mention de la délivrance du brevet: **21.10.2009**
(21) Numéro de dépôt: 99103418.2
(22) Date de dépôt: 23.02.1999
(51) Int. Cl.: G01N 27/327, C12Q 1/00, C12Q 1/56

(54) **Système électrochimique pour la détermination d'un temps de coagulation du sang**
Elektrochemisches System zur Bestimmung der Blutgerinnungszeit
Electrochemical system for the determination of blood coagulation time

(43) Date de publication de la demande: 30.08.2000
(60) Demande divisionnaire: 08171037.8
(73) Titulaire: ASULAB S.A., 2501 Bienne (CH)
(72) Inventeur: Frenkel, Erik Jan, 2009 Neuchâtel (CH); Haeberli, André, 3073 Gümligen (CH); Moresi, Anita, 3008 Bern (CH)
(74) Mandataire: Couillard, Yann Luc Raymond

(56) Documents cités:
- EP-A- 0 737 853
- WO-A-94/16095
- US-A- 4 304 853
- US-A- 5 418 141

## Description

La présente invention a pour objet un système individuel de détermination du temps de coagulation du sang entier, un capteur jetable de petites dimensions comportant un réactif spécifique de l'enzyme thrombine pour permettre une détermination électrochimique et un appareil de mesure électronique permettant de corréler un signal électrique reçu dudit capteur lorsqu'il a été introduit dans l'appareil et qu'une goutte de sang à analyser a été déposée sur celui-ci.

L'invention concerne plus particulièrement un tel appareil de mesure et un capteur permettant de déterminer par ampérométrie le temps de prothrombine (PT) lorsque la composition du réactif déposé sur le capteur comprend un substrat chimique dont une fraction terminale peut être sélectivement coupée par l'enzyme thrombine en libérant un groupe chargé.

Le contrôle du temps de coagulation du sang, c'est-à-dire l'aptitude des composants du sang à former un caillot pour prévenir un risque hémorragique, fait partie des examens de routine, voire d'examens journaliers, dans de nombreuses situations pathologiques acquises, traumatiques ou postopératoires. Il est par exemple nécessaire lors d'un traitement par anticoagulants dans le cas de maladies cardiaques de pouvoir ajuster précisément la posologie du médicament, par exemple la warfarin ou l'héparine, afin d'éviter les risques hémorragiques en cas de surdosage ou au contraire les risques de thrombose si la quantité d'anticoagulants administrée est insuffisante.

Différents paramètres ont été retenus pour effectuer cette détermination, mais le plus usuel est la mesure du temps de prothrombine (PT) après activation, c'est-à-dire le temps au bout duquel on observe la formation d'un caillot avec un échantillon de sang prélevé sur un patient. De telles analyses ont longtemps relevé de la seule compétence d'un personnel spécialisé de laboratoire équipé d'appareils complexes et encombrants. Cela avait l'inconvénient d'obliger le patient à se déplacer, de nécessiter une préparation d'un échantillon de sang prélevé à domicile, par exemple par adjonction de citrate, pour attendre le moment de pouvoir effectuer une analyse en laboratoire.

Les progrès réalisés au niveau de la miniaturisation, notamment par l'emploi de composants électroniques, ont permis depuis un peu plus d'une dizaine d'années de procurer à un patient divers types d'appareils moins encombrants qui lui permettent d'effectuer chez lui une mesure du temps de coagulation. Dans leur majorité ces appareils individuels font appel aux mêmes principes que les appareils de laboratoire, à savoir l'observation directe de la dynamique des érythrocytes dans un échantillon de sang, auquel ont été ajoutés des réactifs usuels de coagulation, lorsqu'il passe d'un état fluide à un état visqueux ou figé.

Selon un premier principe, on mesure le temps au bout duquel un échantillon de sang préparé ne s'écoule plus à travers un tube capillaire, ou à travers l'étranglement calibré d'un tube de plus grand diamètre, supporté par un moyen récepteur à usage unique adaptable à un appareil de mesure. Cet écoulement est généralement forcé au moyen d'un dispositif de pompage pneumatique intégré dans l'appareil de mesure, et la détection du temps au bout duquel se produit la coagulation est généralement détectée par des moyens optiques. Des dispositifs de ce type sont par exemple décrits dans les documents US 3,486,859, US 3,890,098 et US 5,302,348. Un appareil reposant sur ce principe est par exemple celui proposé sous la marque "Hemochron", ou selon une variante plus récente sous la marque "Protime Microcoagulation System" par International Technidyne Corporation (NJ - USA). L'appareil de mesure comporte évidemment une source d'énergie pour alimenter à la fois la partie mécanique (pompe) et le contrôle de fin de coagulation (détection optique). On observera en outre que chaque moyen récepteur de l'échantillon de sang à analyser, jetable après le premier usage est relativement encombrant (environ 3 x 9 cm) et relève d'une technologie de précision (calibration du tube capillaire ou de l'étranglement) qui contribue nécessairement à élever le coût de chaque analyse effectuée.

Selon un deuxième principe, on mesure le temps au bout duquel un échantillon de sang préparé, et déposé dans un coupelle jetable permet par coagulation d'immobiliser un objet magnétique mis en mouvement par un champ magnétique tournant, la détection du phénomène de coagulation étant là encore le plus souvent effectuée par des moyens optiques. Le document US 3,967,934 décrit déjà ce principe dans lequel le container destiné à recevoir l'échantillon contient une bille ferromagnétique. Un tel dispositif est par exemple utilisé dans l'appareil diffusé par Nycomed Pharma (Oslo Norvège) sous la marque "Trombotrack". Selon une autre variante, décrite par exemple dans le document US 5,154,082, la bille est remplacée par des particules ferromagnétiques, également soumises à un champ électromagnétique. Cela a permis de réaliser un réactif sous forme sèche, déposée sur un support, la mobilité des érythrocytes étant toujours détectée par des moyens optiques. Un dispositif du type précédent correspond par exemple à un appareil de Boehringer Mannheim (Allemagne) vendu sous la marque Coaguchek. Les produits proposés correspondant à ce deuxième principe présentent les mêmes inconvénients que ceux déjà mentionnés pour les appareils selon le premier principe à l'exception peut-être du coût moindre des moyens récepteurs de l'échantillon de sang pour l'appareil Coaguchek.

Des tests comparatifs effectués avec les méthodes et dispositifs de l'art antérieur précité ("Home Prothrombin Estimation" Angelida Bernardo et al., Thrombosis, Embolism and Bleeding, ch. 3.5 - E. G. Butchart et E. Bodnar ICR Publishers 1992) ont montré que le suivi médical d'un patient à domicile était au moins aussi satisfaisant que celui d'un patient en milieu hospitalier, mais que des résultats fiables et reproductibles ne pouvaient être obtenus qui si le patient avait suivi un apprentissage plus ou moins long. Les appareils de cet art antérieur sont évidemment prévus pour un usage domestique, sont facilement déplaçables mais restent encore relativement trop volumineux pour qu'un patient puisse le garder à demeure sur lui, par exemple dans une poche de vêtement, lors de ses déplacements. Il est sans doute également souhaitable, pour la fiabilité de mesures qui dépendent de dispositifs à la fois optiques et électromécaniques, que lesdits appareils subissent le moins de déplacements possible.

On observera enfin que, selon l'un ou l'autre des principes ci-dessus, la source d'énergie électrique nécessaire pour alimenter les dispositifs optiques et électromécaniques doit être relativement importante lorsqu'elle est autonome (pile ou batterie) mais qu'elle n'intervient jamais directement dans la mesure du temps de coagulation.

On peut toutefois signaler un brevet US 3,674,012 de 1972 qui décrit une seringue dont la paroi interne comporte une succession d'électrodes permettant de mesurer la variation d'impédance du milieu, au moyen d'un oscilloscope connecté à ladite seringue, au fur et à mesure que le phénomène de coagulation progresse. Un tel dispositif ne repose encore que sur la variation des propriétés du milieu en cours de coagulation et n'est manifestement pas destiné à un usage individuel.

Le dispositif décrit dans le document EP 0 679 193, permettant entre autre de déterminer le temps de prothrombine, comporte deux électrodes qui n'interviennent dans ladite détermination que pour détecter, à partir d'un signal représentatif de la variation de résistance entre les électrodes, la présence d'un échantillon de sang sur le moyen récepteur, et n'ont aucun rôle direct pour mesurer une durée. Dans ce dispositif la mesure de la durée est effectuée par une détermination photométrique de la fluorescence du milieu à partir d'un substrat oligopeptidique ayant la Rhodamine comme groupe terminal pouvant être libérée en étant coupée par l'enzyme thrombine. Un substrat de ce type est par exemple décrit dans le document US 4,557,862. Une méthode colorimétrique utilisant un substrat un peu différent, ayant comme chromatophore la p-nitroaniline correspond par exemple au produit de Nycomed Pharma commercialisé sous la marque "NYCOTEST-CHROM".

Ces méthodes colorimétriques présentent l'avantage de ne plus faire appel à des moyens mécaniques ou électromécaniques, mais il est en contrepartie nécessaire d'avoir une étape supplémentaire de centrifugation ou d'ultrafiltration à travers une membrane, comme proposé dans le document EP 0 679 193, pour éliminer les érythorcytes afin d'effectuer la détection seulement sur la partie plasmatique. Dans le document EP 0 182 373 l'élimination des érythrocytes avant mesure colorimétrique s'effectue au moyen d'une membrane de non-tissé intégrée dans la construction du capteur. Ces méthodes ont de plus l'inconvénient d'exiger un appareil de mesure ayant des moyens de détection optique de grande précision, donc un appareil relativement fragile et peu transportable, et de demander un temps de développement de la couleur relativement long, généralement supérieur à 5 minutes, avant de pouvoir effectuer la mesure.

Une méthode plus simple dans son principe, qui repose sur une mesure électrochimique, est décrite dans le document US 4,304,853. Cette méthode consiste à introduire dans une cellule de mesure comportant au moins deux électrodes, un échantillon de sang citraté et un substrat oligopeptique qui doit être maintenu dans un milieu solvant tel que le diméthylsulfoxyde (DMSO). Le substrat se compose d'un enchaînement d'amino-acides ayant l'arginine comme amino-acide terminal, lié à un "leaving group" électroactif et dont un hydrogène de l'amino-acide initial peut être remplacé par un groupe protecteur, ces amino-acides, "leaving groups" et groupes protecteurs étant choisis dans des listes très limitatives. En raison de la complexité du phénomène de coagulation due à une cascade de réactions, la présence d'un solvant peut interférer avec les produits intermédiaires conduisant à la libération de thrombine et nuire ainsi à la précision de la mesure.

Dans une demande de brevet internationale PCT intitulée "Oligopeptidderivate", déposée ce même jour par la société Pentapharm AG (Bâle-Suisse), une seléction particulière des maillons constitutifs du substrat en particulier des amino-acides et du Leaving group, permet d'effectuer, au moyen d'une cellule de mesure de même nature que précédemment, une détermination d'un temps de coagulation d'un échantillon de sang en milieu liquide, sans qu'il soit nécessaire d'ajouter un solvant ou un co-solvant pour maintenir le substrat en solution. Même si l'utilisation de ces nouveaux substrats présente un avantage certain, l'utilisation d'une cellule de mesure, avec les manipulations que cela nécessite, ne permet pas encore de procurer à un patient, qui voudrait contrôler lui-même sont temps de coagulation, un appareil facilement transportable et simple à utiliser.

Le système selon l'invention a pour but de pallier les inconvénients existant encore dans les systèmes de l'art antérieur en procurant un appareil et des capteurs d'encombrement très réduit grâce à l'utilisation de réactifs spécifiques et d'une méthodologie différente de ce qui était antérieurement utilisé pour la détermination individuelle d'un temps de coagulation.

A cet effet l'invention a pour objet un système de mesure électrochimique d'une valeur représentative d'un temps de coagulation d'une goutte de sang entier caractérisé en ce qu'il comprend :
- un capteur électrochimique en forme de languette de petites dimensions supportant au moins une électrode référence et une électrode de travail sur laquelle est immobilisé un réactif spécifique dont la composition incorpore au moins un substrat chimique dont un maillon terminal peut être coupé par l'enzyme thrombine pour donner des groupes chargés (LG), et
- un appareil de mesure dont le circuit électronique, alimenté par une source d'énergie, permettant d'imposer entre les électrodes du capteur un courant électrique variable ou non ou une différence de potentiel variable ou non, de traiter le signal électrique résultant de la migration des groupes chargés (LG), de corréler ledit signal avec une valeur représentative du temps de coagulation et d'afficher ladite valeur sur un écran d'affichage dudit appareil de mesure.

Le réactif spécifique immobilisé sur l'électrode de travail comprend en outre une thromboplastine et un milieu tampon.

Le substrat chimique dont un maillon terminal peut être coupé par l'enzyme thrombine pour libérer des groupes chargés LG doit avoir un site stéréospécifique de l'enzyme thrombine. Un substrat chimique approprié est par exemple constitué par un dérivé oligopeptidique et plus particulièrement par un dérivé oligopeptidique ayant pour amino acide terminal l'arginine (arg) liée à un groupe pouvant être coupé par la thrombine pour donner des groupes chargés (LG). Selon un mode de réalisation préféré ces groupes libérables par la thrombine sont choisis parmi les groupes amino-aryl ou amino-hétéroaryl éventuellement substitués, la liason avec l'arginine s'effectuant par l'une de leurs fonctions amines

L'invention sera exposée plus en détails en référence aux dessins annexés, dans lesquels :
- la figure 1 représente en perspective un appareil de mesure et un capteur d'un système selon l'invention par la détermination d'un temps de coagulation du sang,
- la figure 2 est un schéma du processus conduisant à un signal électrique exploitable par l'appareil de mesure.
- les figures 3a à 3e représentent des courbes de variation du signal en fonction du rapport PC/PS.
- la figure 4 représente des courbes de variation du signal en fonction du milieu tampon.
- la figure 5 représente des courbes de variation du signal en fonction du mode de séchage.
- la figure 6 représente des courbes montrant la spécificité du substrat.

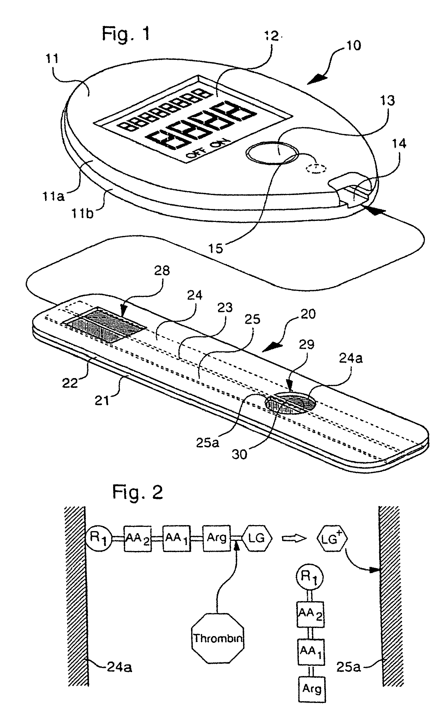

A la figure 1 on a représenté, sensiblement à l'échelle 1/1, un appareil de mesure 10, et sensiblement à l'échelle 3/1, un capteur électrochimique 20 en forme d'une languette d'environ 40 mm de long et 8 mm de large.

L'ensemble formé par l'appareil et un conditionnement de plusieurs capteurs a donc un encombrement comparable à celui de n'importe quel autre appareil portable, ce qui permet à un patient de l'avoir sur lui lors de tous ses déplacements.

L'appareil de mesure 10 et le capteur électrochimique 20 sont issus d'une même technologie comparable à celle utilisée pour le dosage du taux de glucose, par exemple par une méthode ampérométrique telle que décrite dans les brevets US 5,378,628, US 5,532,602 et EP 0 787 984.

L'appareil de mesure 10, en tant que tel, comprend un boîtier 11 de forme ovoïde dans l'exemple représenté, construit par assemblage de deux éléments plastiques moulés 11 a, 11 b, dont l'élément supérieur 11 a comporte une fenêtre pour un écran d'affichage 12, et une ouverture pour un bouton de commande 13 permettant d'accéder à différents modes d'affichage.

Le circuit électronique inclus dans le boîtier est une adaptation des circuits utilisés pour le dosage du glucose, par exemple par ampérométrie comme indiqué précédemment et n'en diffère que par un paramétrage différent du signal électrique afin d'afficher un temps de coagulation.

L'appareil de mesure 10 comprend également une fente 14, disposée sensiblement entre les deux parties 11 a, 11 b du boîtier 11, dans laquelle on introduit une portion du capteur 20, qui comporte la zone de contact 28. L'autre portion du capteur 20 comportant une zone de mesure 29 destinée à recevoir un échantillon de sang, restera à l'extérieur de l'appareil 10 pendant tout le temps de la mesure. Dans la plupart des appareils de mesure de l'art antérieur l'échantillon est au contraire introduit à l'intérieur de l'appareil dans une enceinte thermostatée généralement à 37°C, ce qui conduit à une consommation d'énergie supplémentaire.

Dans le système selon l'invention, dans lequel l'échantillon n'est pas introduit dans une enceinte thermostatée, il est facile de prévoir, si nécessaire, une sonde thermique 15 sur l'appareil à proximité de la fente 14 pour adapter le paramétrage du signal électrique en fonction de la température ambiante, avec une consommation d'énergie bien inférieure à celle nécessaire pour thermostater une enceinte.

Le capteur électrochimique 20 représenté à la figure 1 est du type de ceux utilisés pour la détermination du taux de glucose par ampérométrie comme indiqué précédemment. Il comprend un support plastique mince 21, réalisé par exemple en PET, supportant sur toute sa longueur deux collecteurs de courant 24, 25 séparés par un petit espace 23 qui les isole électriquement.

Le support 21 et les collecteurs 24, 25 sont recouverts d'un revêtement isolant 22 dans lequel sont découpées près de chaque extrémité, par exemple par estampage, deux fenêtres 28, 29 laissant apparaître des portions de collecteurs 24, 25. Une première fenêtre 28 permet de connecter électriquement le capteur 20 à l'appareil de mesure 10, tandis que la deuxième fenêtre 29 constitue la zone de mesure, les portions visibles des collecteurs formant respectivement l'électrode de travail 24a et l'électrode de référence 25a.

L'électrode de travail 24a est par exemple réalisée par laminage d'une mince bande de platine et l'électrode de référence 25a par laminage d'une mince bande d'argent ultérieurement chlorurée pour former en même temps une électrode de référence. Il est également possible de prévoir de façon séparée dans la zone de mesure 29, une électrode de travail, une électrode de référence et une contre électrode. L'électrode de travail est revêtue d'un réactif spécifique 30 décrit plus en détail ci-après pour permettre la mesure du temps de prothrombine (PT).

Le réactif spécifique est immobilisé sous une forme sèche à partir d'une composition comprenant dans l'exemple ici décrit au moins une tromboplastine, un milieu tampon et un substrat oligopeptidique ayant une configuration stéréospécifique d'un site de l'enzyme thrombine pour permettre une coupure d'une partie terminale chargée généralement désignée par "leaving group" (LG). Dans les expérimentations rapportées ci-après le substrat utilisé est caractérisé en ce que le le dérivé oligopéptidique ou l'un de ses sels comprend outre l'arginine au moins un autre radical amino-acide choisi parmi l'acide 2-aminobutyrique (Abu), l'alanine (Ala), la 3-cyclohexylalanine (Cha), la 2-cyclohexylglycine (Chg), la phenylalanine (Phe), l'acide pipécolique (Pip), la proline (Pro), la valine (Val), et la glycine (Gly) lesdits amino-acides pouvant être sous forme L, D ou DL.:

Ces oligopeptides et la façon de les obtenir sont décrits plus en détails dans la demande de brevet de la société Pentapharm AG précitée, et dans laquelle le grand intérêt de ces oligopeptides pour la détermination d'un temps de coagulation a été établi de façon expérimentale en milieu liquide au moyen d'une cellule de laboratoire. Il est donc précisé que la présente invention ne concerne pas lesdits oligopeptides en tant que tels, mais les conditions d'optimisation de compositions qui permettent, lorsqu'elles sont déposées sous forme sèche sur un capteur électrochimique, d'obtenir un signal électrique qui soit suffisant pour être traité, qui permette une bonne linéarisation et dont le temps de réponse soit suffisamment court.

De même le "leaving group" (LG) qui doit, dans les conditions de l'invention, à la fois facilement réagir avec la fonction acide de l'arginine (Arg), pouvoir être coupé par la thrombine et posséder une charge suffisante, est choisi de préférence parmi les dérivés d'aniline, de quinolylamine et de naphtylamine éventuellement substitués par un ou plusieurs substituants choisis parmi un halogène, un radical hydroxy, amino, nitro, alkyl, alkoxy, alkanoyl, anilino, et aminophenyl éventuellement substitué.

Des expériences ont montré qu'un substrat donné peut permettre de détecter un signal électrique en milieu liquide, tout en étant inopérant ou peu efficace lorsque le même substrat est utilisé sous une forme sèche déposée sur l'électrode de travail d'un capteur électrochimique. Un des facteurs pouvant expliquer cette différence est le fait que l'oligopeptide doit être immobilisé sur l'électrode de travail par l'extrémité de sa chaîne opposée à celle comportant le "leaving group" LG. Ce résultat peut être atteint par le premier amino-acide de la chaîne oligopeptique, mais de préférence un hydrogène de sa fonction amino terminale est remplacé par un "groupe protecteur" R₁ choisi parmi les acides Boc (terbutoxycarbonyl), Tos (para-toluenesulfonique), t-Bups (ter-butylphenylsulfonyl), Mes (methylsulfonyl), Naps (2-naphtylsulfonyl), Bzo (benzoyl)
Z (benzyloxycarbonyl), isopropylsulfonyl, camphosulfonyl.

Selon un mode de réalisation préféré, on choisit le groupe protecteur, les α-amino-acides et leur enchaînement, ainsi que les "leaving-group" de façon à avoir les substrats oligopeptidiques ci-après :
- Z-Gly-Pro-Arg-3-chloro-4-hydroxyanilide
- Tos-Gly-Pro-Arg-3-chloro-4-hydroxyanilide
- Boc-(D)-Chg-Gly-Arg-3-chloro-4-hydroxyanilide
- H-(D)-Chg-Gly-Arg-3-chloro-4-hydroxyanilide
- Z-Gly-Pro-Arg-2-chloro-4-hydroxyanilide
et leurs sels minéraux ou organiques compatibles.

Les oligopeptides entrant dans la composition du réactif spécifique sont généralement utilisés sous forme de l'un de leurs sels, formé par exemple avec l'acide chlorhydrique (HCI), l'acide acétique ou l'acide trifluoroacétique (TFA).

Tous les réactifs permettant de déterminer un temps de coagulation, tel que le temps de prothrombine, contiennent dans leur composition une tromboplastine. Dans les méthodes d'analyses les plus récentes on utilise une tromboplastine reconstituée afin d'éviter les inconvénients des substances extraites de tissus vivants, tels que les risques de contamination et les interférences avec les médicaments ou leurs métabolites véhiculés par le sang. Il s'agit par exemple du produit Innovin® (Dade Int. III. USA), faisant notamment l'objet du brevet EP 0 565 665, dont la composition comprend 25 à 35% d'une phosphatidylsérine (PS) à polarité négative et 65 à 75% d'une phosphatidylcholine (PC) à polarité positive.

De façon surprenante on a observé que le système selon l'invention permettait d'obtenir une meilleure détermination (hauteur du signal, linéarité) du temps de prothrombine (PT) en utilisant au contraire un facteur tissulaire avec des phospholipides présentant un rapport PS/PC inversé, dans lequel la quantité de phospholipides négatifs est supérieure à la quantité de phospholipides positifs.

Le réactif spécifique peut incorporer dans sa composition en tant qu'activateur un sel de calcium tel que Ca Cl₂. Toutefois, les conditions d'optimisation obtenues par ailleurs font qu'il n'est pas toujours nécessaire d'incorporer un sel de calcium étant donné que la concentration en Ca⁺⁺ dans le sang entier est déjà suffisant pour l'activation.

Comme dans la plupart des réactions biologiques il est nécessaire de maintenir le milieu à un pH déterminé et d'avoir une force ionique suffisante. Parmi tous les milieux tampons bien connus de l'homme de l'art, le tampon Hepes (acide N-2-hydroxyéthylpipérazine-N'-2-aminoethane sulfonique) est celui qui s'est révélé le plus approprié lorsqu'on utilise des substrats oligopeptides de la société Pentapharm, comme cela sera montré par la suite.

On observera enfin que l'immobilisation du réactif spécifique sur l'électrode de travail à partir d'une composition liquide ou pâteuse peut être obtenue, sans autres additifs, par les techniques connues de l'homme de l'art. La technique de lyophilisation s'est toutefois révélée comme étant la plus satisfaisante, comme cela sera montré plus loin.

La figure 2 est une représentation schématique de la réaction chimique qui permet de générer un courant entre les électrodes 24a et 25a qui sont reliées par un circuit électronique de détection non représenté. Le substrat, qui peut être représenté schématiquement par la formule R₁-AA₂-AA₁-Arg-LG dans laquelle AA₁ et AA₂ représentent d'autres amino-acides tels qu'indiqués précédemment, est lié à l'électrode de travail 24a par le groupe R₁ qui oriente l'oligopeptide, et dont l'autre extrémité de la chaîne comporte un "leaving group" LG tel que défini précédemment. Dans la partie gauche du schéma, on voit que l'enzyme thrombine coupe sélectivement la liaison entre l'arginine et ledit "leaving group" LG. Dans la partie droite du schéma, on voit que le "leaving group" libéré peut migrer vers l'électrode 25a et engendrer un courant qui sera proportionnel au nombre de "leaving group" LG libérés, et donc à la quantité de thrombine formée dans l'échantillon.

Les expérimentations rapportées ci-après montrent également que les substrats oligopeptides utilisés sont sélectivement coupés par l'enzyme thrombine à l'exclusion de toute autre enzyme présente dans le sang entier.

Dans les expériences rapportées ci-après on a effectué des mesures potentiostatiques par chronoampérométrie au moyen d'un potentiostat PGP 201 connecté au Software Vm, de Radiometer (Copenhague) et permettant d'obtenir les conditions ci-après :

| | | | |
|---|---|---|---|
| Potentiel 1: 0mV | Temps 1 | : 1 sec. | |
| Potentiel 2: 300mV | Temps 2 | : 5 min. | |
| Nombre de cycles | : 1 | Fréquence de mesure | : 1/sec. |
| Courant minimum | : -50mA | Courant maximum | : 50mA |
| Sensibilité minimum | : 1µA | | |

La surface de l'électrode de travail du capteur utilisé est de 0,035 cm² et la quantité d'échantillon, goutte de sang ou solution de référence, est de 10µl. On commence l'enregistrement des mesures 10 secondes après le dépôt de l'échantillon. L'oligopeptide utilisé, ci-après désigné selon l'usage par "substrat", choisi parmi ceux précédemment indiqués, est :
Tos-Gly-Pro-Arg-3-chloro-4-hydroxyanilide, 2HCI.

### Expérience 1 : Influence du rapport PC/PS

On sait que le facteur tissulaire humain reconstitué et activé par une phosphatidylserine (PS) et une phosphatidylcholine (PC) dans une proportion 30/70, active la coagulation dans le plasma en présence de CaCl₂. Cette composition permet aussi l'activation dans le sang entier, mais la recherche d'une optimalisation s'est avérée nécessaire pour obtenir un signal amperométrique suffisamment élevé. Pour cela on a effectué des séries de mesures en faisant varier le rapport PS/PC, comme indiqué ci-après.

Dans 1000µl d'une solution de "tampon TRIS" (triméthylol aminoéthane 50mM, NaCI/100mM, NaN₃ 0,02 %) contenant 2,5mg du sel de sodium de l'acide déoxycholique on a dissous 5 µmol de PC en utilisant un bain à ultrasons pendant 5 minutes pour obtenir une première solution-mère. De même on a préparé une deuxième solution-mère contenant 5 µMol de PS. On a ensuite mélangé les deux solutions mères pour obtenir une concentration totale en phospholipide (PS + PC) de 2,5 mg par 1000 µl, après ajustement avec la solution de dilution précitée, de façon à avoir la gamme suivante :

| | | | |
|---|---|---|---|
| a - PC | 100% - | PS | 0% |
| b - PC | 75% - | PS | 25% |
| c - PC | 50% - | PS | 50% |
| d - PC | 25% - | PS | 75% |
| e - PC | 0% - | PS | 100% |

lesdits pourcentages étant exprimés en mol%.

On a d'autre part mélangé 10µl de facteur tissulaire à une concentration de 164mg/ml (Recombinant human tissue factor de American Diagnostica 4500) avec 20 µl d'eau distillée, puis ajouté 10µl d'une composition PS/PC précédente en complétant jusqu'à 600 µl avec une solution de TRIS contenant du BSA (Bovine Serum Albumine), à raison de 1mg/ml de BSA pour 260 µl, que l'on soumet à agitation pendant 2 minutes. On dilue ensuite 220 µl de cette solution avec solution de TRIS jusqu'à obtenir un volume final de 1100 µl. On répète l'opération précédente avec les autres compositions PS/PC de façon à avoir une gamme de 5 solutions de tromboplastines différentes.

Finalement, en utilisant un bain à ultrasons pendant 5 minutes, on dissout dans chacune des solutions précédentes le substrat à une concentration de 0,33mg/ml (0,5mM/l), de façon à obtenir 5 solutions de substrat, c'est-à-dire 5 réactifs spécifiques dans lesquels le rapport PC/PS varie dans les proportions indiquées précédemment.

Les capteurs électrochimiques soumis à expérience sont enfin préparés en déposant 10µl de réactif spécifique sur l'électrode de travail, puis en effectuant un séchage dans un four à 30° pendant 2h.

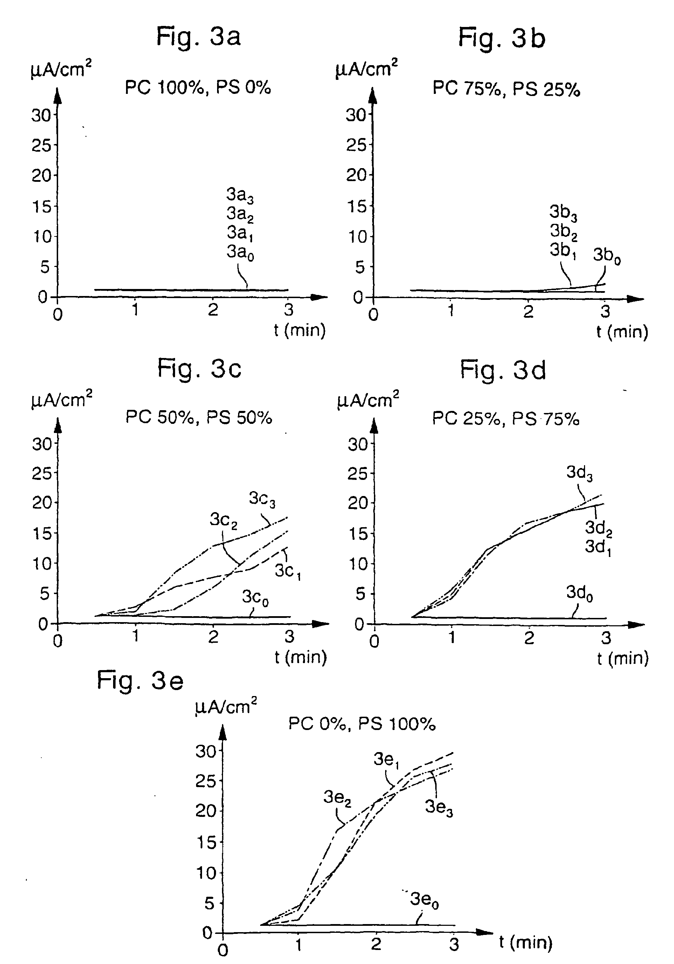

Les figures 3a à 3e sont des courbes représentant la densité de courant exprimée en µA/cm² en fonction du temps exprimé en minutes, les résultats enregistrés pour trois essais différents (3a1, 3a2, 3a3; 3b1, 3b2... etc) en référence à un essai témoin (3a0, 3b0, 3c0, 3d0, 3e0) contenant 10µl de tampon TRIS au lieu de 10µl de sang entier. Comme on le voit à la figure 3a, les courbes 3a0, 3a1, 3a2 et 3a3 sont confondues, c'est-à-dire qu'aucun signal électrique n'est obtenu lorsque le rapport PC/PS est de 100/0. Lorsque le rapport PC/PS est de 75/25, ce qui correspond au rapport préféré de la composition Innovin®, on voit sur la figure 3b que la densité de courant est très faible et se différencie à peine de la courbe témoin 3b0. Lorsque PC et PS sont en égale quantité (Fig. 3c) on obtient une densité de courant significative mais une trop grande dispersion des mesures. Avec un rapport PC/PS de 25/75 on voit à la figure 3d que les courbes 3d1, 3d2, 3d3 sont pratiquement confondues, c'est-à-dire qu'on obtient une bonne reproductibilité des mesures et une linéarité satisfaisante. En utilisant PS seul (Fig. 3d), le signal est plus élevé, mais la reproductibilité peut sembler moins satisfaisante. Ces expériences montrent que la composition préférée doit avoir de 65% à 100% de phosphatidylsérine (PS).

De plus on observera que les résultats ci-dessus ont été obtenus sans qu'il ait été nécessaire d'ajouter du CaCl₂, étant donné que la concentration en ion calcium dans le sang entier s'est avérée suffisante.

D'autres expériences ont montré que l'addition de phosphatidyléthanolamine (PE) n'apportait pas d'amélioration aux mesures, et qu'il n'était pas possible d'obtenir une activation uniquement avec le facteur tissulaire, c'est-à-dire sans phospholipides.

### Expérience 2 : Influence du milieu tampon

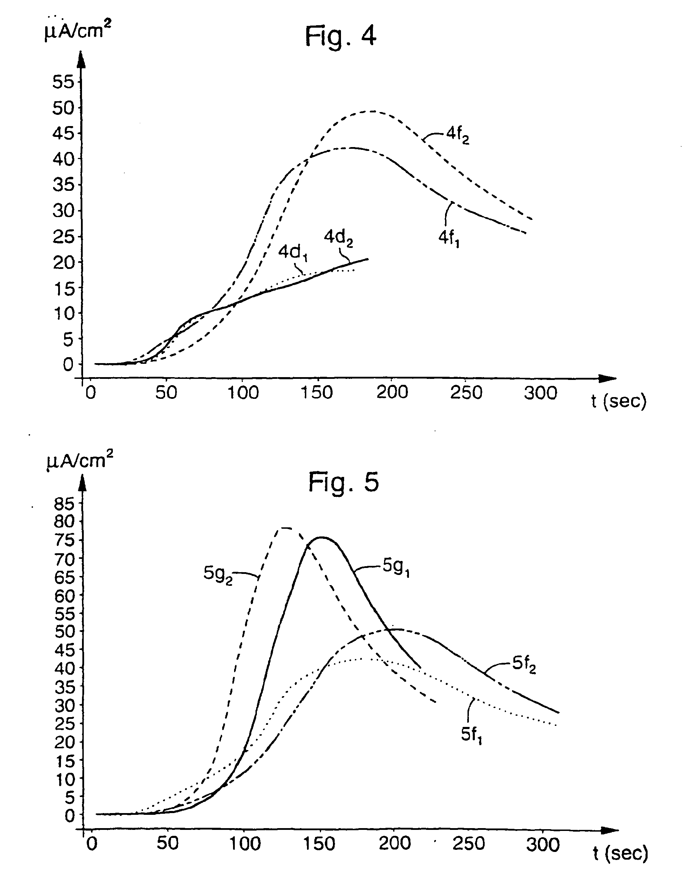

Selon le même processus que celui décrit pour l'expérience 1, on a préparé une première solution de substrat (d) contenant PC-PS dans le rapport 25/75 avec le tampon TRIS comme milieu tampon, et une deuxième solution de substrat (f) dans laquelle le tampon TRIS est remplacé par le tampon Hepes, puis on a effectué avec chaque solution de substrat, en employant un échantillon de sang d'un même patient deux mesures correspondant respectivement aux courbes 4d1, 4d2 (TRIS) et 4f1, 4f2 (Hepes) de la figure 4. Comme on le voit, on obtient un signal beaucoup plus élevé avec le tampon Hepes, qui peut donc être retenu comme milieu tampon préféré.

### Expérience 3 : Influence du mode de séchage

Dans la présente expérience on a comparé la forme du signal obtenu lorsque le réactif spécifique est séché par lyophilisation avec celui obtenu par séchage au four à 30°C pendant 2h. comme c'était le cas dans les expériences précédentes. Le réactif spécifique est le même que celui de l'exemple 2 avec le tampon Hepes. En employant l'un ou l'autre mode de séchage on effectue chaque fois deux mesures correspondant respectivement aux courbes 5f1, 5f2 (séchage au four) et 5g1, 5g2 (lyophilisation). On peut observer que la technique de lyophilisation permet d'obtenir un signal plus élevé, dans un temps beaucoup plus court. Cela peut être expliqué par le fait que la lyophilisation permet d'obtenir un recouvrement plus homogène de la surface des électrodes que celui obtenu avec un séchage au four.

D'autres expériences réalisées uniquement avec le substrat en milieu tampon et en utilisant des adjuvants qui permettaient d'optimiser le signal dans le cas d'un capteur de glucose, ont montré que l'utilisation d'une poudre de carbone pour augmenter la surface spécifique n'avait aucun effet favorable, du fait que le courant de base dû à la poudre de carbone interférait avec le courant à mesurer.

### Expérience 4 : Spécificité du réactif spécifique vis-à-vis de l'enzyme thrombine

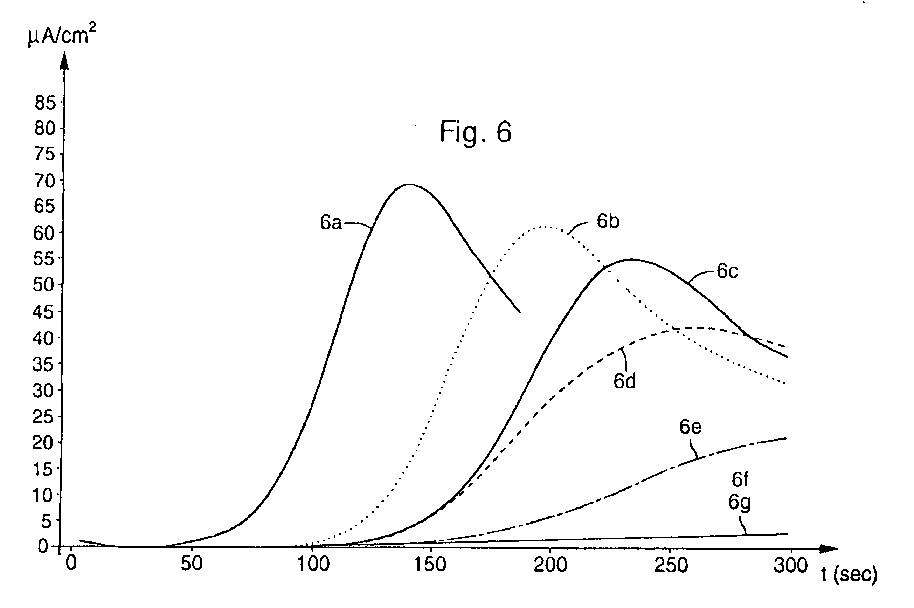

Etant donné que le sang entier contient de très nombreux composants, la présente expérience permet de montrer que seule l'enzyme thrombine est capable de couper le substrat du réactif spécifique. L'expérience est réalisée dans les conditions d'optimisation définies par l'expérience 3, (à savoir PC/PS dans le rapport 25/75, tampon Hepes et lyophilisation) en partant d'une dilution à parts égales de sang et de solution tampon et en inhibant progressivement l'enzyme thrombine par des concentrations croissantes de r-hirudin (r-hirudin 2000 ATU/ampoule, disponible chez Pentapharm AG Bâle-Suisse) qui remplacent la solution tampon, en ayant préalablement vérifié que le facteur Xa n'était pas inhibé par la r-hirudin. A la figure 6, la courbe 6a est une courbe de contrôle sans r-hirudin et les courbes 6b à 6g les courbes des mesures effectuées avec respectivement 12,5 ATU/ml; 25ATU/ml; 37,5 ATU/ml; 50 ATU/m.; 100 ATU/ml; 1000 ATU/ml.

Comme on le voit la hauteur du signal diminue au fur et à mesure que la concentration en r-hirudin augmente jusqu'à devenir pratiquement nul à partir de 100 ATU/ml. On peut donc en conclure que le système selon l'invention permet au substrat d'être sélectivement coupé par la thrombine, et donc de permettre une détermination sélective de cette enzyme.

Comme on le voit en se référant plus particulièrement aux courbes 6a, 6b et 6c, la valeur maximum est sensiblement proportionnelle à là diminution de l'enzyme thrombine dans l'échantillon, ce qui permet d'envisager un paramétrage linéaire. Dans la pratique, on ne prend pas la valeur maximum, mais la valeur correspondant au point d'inflexion de la partie ascendante de la courbe, c'est-à-dire le point pour lequel la vitesse de libération de la thrombine est maximum. Dans la pratique on utilise des fenêtres de détection de 15 secondes et on retient la valeur comprise entre deux fenêtres de détection pour lesquelles on observe le passage d'une augmentation du sens de la pente à une diminution du sens de la pente.

## Revendications

1. Capteur électrochimique (20) pour la mesure d'une valeur représentative (PT) d'un temps de coagulation d'une goutte de sang entier, **caractérisé en ce qu'**il comprend :
- un support (21, 22) isolant en forme de languette supportant au moins une électrode de référence (25a) et une électrode de travail (24a), et comportant une zone de mesure (29) destinée à recevoir un échantillon de sang entier ;
- l'électrode de travail (24a) étant revêtue d'un réactif spécifique (30) sous forme sèche dont la composition incorpore au moins une thromboplastine et un substrat comportant un dérivé oligopeptidique ayant une configuration stéréospécifique d'un site de l'enzyme thrombine pour permettre la coupure par cette dernière d'une partie terminale chargée (LG) dudit substrat.

2. Capteur électrochimique (20) selon la revendication 1, **caractérisé en ce que** le réactif spécifique (30) comprend en outre un milieu tampon afin de maintenir une force ionique suffisante.

3. Capteur électrochimique (20) selon la revendication 2, **caractérisé en ce que** le milieu tampon est du type HEPES afin d'obtenir un signal plus élevé.

4. Capteur électrochimique (20) selon l'une des revendications 1 à 3, **caractérisé en ce que** ladite au moins une thromboplastine du réactif spécifique (30) comprend une composition dans laquelle la quantité de phospholipides négatifs est supérieure à la quantité de phospholipides positifs.

5. Capteur électrochimique (20) selon la revendication 4, **caractérisé en ce que** le taux de phospolipides négatifs, tel que la phosphatidylsérine (PS), est supérieur à 65%.

6. Capteur électrochimique (20) selon l'une des revendications précédentes, **caractérisé en ce que** le réactif spécifique (30) comprend en outre dans sa composition un activateur comportant un sel de calcium tel que CaCl₂.

7. Capteur électrochimique (20) selon l'une des revendications précédentes, **caractérisé en ce que** ledit dérivé oligopeptidique comprend pour amino-acide terminal l'arginine (Arg) liée à au moins un autre radical amino-acide choisi parmi l'acide 2-aminobutyrique (Abu), l'alanine (Ala), la 3-cyclohexylalanine (Cha), la 2-cyclohexylglycine (Chg), la phenylalanine (Phe), l'acide pipecolique (Pip), la proline (Pro), la valine (Val) et la glycine (Gly), lesdits amino-acides pouvant être sous forme L, D ou DL.

8. Capteur électrochimique (20) selon la revendication 7, **caractérisé en ce que** ladite charge (LG) est reliée à l'arginine (Arg) et comporte un groupe amino-aryl ou amino-hétéroaryl (LG) choisi parmi les dérivés d'aniline, de quinolylamine et de naphtalamine éventuellement substitués par un ou plusieurs substituants choisis parmi un halogène, un radical hydroxy, amino, nitro, alkyl, alkoxy, alkanoyl, anilino et aminophényl éventuellement substitué.

9. Capteur électrochimique (20) selon l'une des revendications précédentes, **caractérisé en ce que** ledit substrat comportant un dérivé oligopeptidique comporte à l'extrémité opposée à la charge (LG) un groupe protecteur (R₁) afin d'immobiliser ledit substrat sur l'électrode de travail (24a).

10. Capteur électrochimique (20) selon la revendication 9, **caractérisé en ce que** le groupe protecteur (R₁) est choisi parmi les acides Boc (ter-butoxycarbonyl), Tos (para-toluenesulfonique), t-Bups (ter-butylphenylsulfonyl), Mes (methylsulfonyl), Naps (2-naphtylsulfonyl),Bzo (benzoyl) Z (benzyloxycarbonyl), isopropylsulfonyl, camphosulfonyl.

11. Capteur électrochimique (20) selon l'une des revendications précédentes, **caractérisé en ce que** le dérivé oligopéptidique consiste en un sel formé avec l'acide chlorhydrique (HCl), l'acide acétique ou avec l'acide trifluoroacétique (TFA).

12. Capteur électrochimique, (20) selon l'une quelconque des revendications précédentes, **caractérisé en ce que** ledit substrat comportant un dérivé oligopeptidique est choisi parmi :
- Z-Gly-Pro-Arg-3-chloro-4-hydroxyanilide
- Tos-Gly-Pro-Arg-3-chloro-4-hydroxyanilide
- Boc-(D)-Chg-Gly-Arg-3-chloro-4-hydroxyanilide
- H-(D)-Chg-Gly-Arg-3-chloro-4-hydroxyanilide
- Z-Gly-Pro-Arg-2-chloro-4-hydroxyanilide
et leurs sels minéraux ou organiques compatibles.

13. Appareil de mesure électronique portable (10) pour la détermination du temps de prothrombine (PT) d'une goutte de sang entier déposée sur une zone de mesure (29) d'un capteur (20) conforme à l'une quelconque des revendications 1 à 12 connecté audit appareil, **caractérisé en ce qu'**il comprend :
- un boîtier (11) logeant une source d'énergie et permettant de connecter ledit capteur et
- un circuit électronique permettant de moduler une tension ou une intensité entre les électrodes (24a, 25a) dudit capteur, d'interpréter un signal représentatif du temps de prothrombine (PT), et de afficher ladite valeur sur un écran (12).

14. Appareil de mesure (10) selon la revendication 13, **caractérisé en ce que** la source d'énergie est une source d'énergie autonome telle qu'une pile ou un accumulateur.

15. Appareil de mesure (10) selon la revendication 13 ou 14, **caractérisé en qu'**il comporte en outre des moyens de mesure de température (15) à proximité de la zone de connexion (14) dudit capteur afin d'adapter le paramétrage dudit signal électrique en fonction de la température ambiante.

16. Méthode de détermination ampérométrique du temps de prothrombine (PT) d'une goutte de sang entier déposée dans une zone de mesure (29) d'un capteur électrochimique (20) conforme à l'une des revendications 1 à 12 introduit dans un appareil de mesure électronique (10) conforme à l'une des revendications 13 à 15, **caractérisée en ce que** le circuit électronique dudit appareil de mesure permet de :
- détecter la présence d'une goutte de sang pour établir entre les électrodes (24a, 25a) une tension déterminée;
- enregistrer une courbe donnant la variation du courant en fonction du temps;
- déterminer au moyen de fenêtres de mesure le point d'inflexion de ladite courbe qui correspond à la vitesse maximale de libération de la thrombine;
- corréler, par exemple au moyen d'une table d'étalonnage, les coordonnées dudit point avec une valeur de temps de prothrombine (PT), et
- afficher ladite valeur sur un écran d'affichage (12).

## Claims

1. Electrochemical sensor (20) for measuring a representing value (PT) of the coagulation time of a drop of whole blood **characterised in that** it includes:
- an insulating support (21, 22) in the shape of a strip holding at least a reference electrode (25a) and a working electrode (24a), and comprising a measuring zone (29) intended to receive a whole blood sample;
- the working electrode (24a) being coated with a dry specific reagent (30) that composition incorporate at least one thromboplastin and a substrate comprising an oligopeptidic derivative having a stereospecific configuration of a site of the thrombin enzyme to allowing cutting off by the latter of a terminal charged portion (LG) of said substrate.

2. Electrochemical sensor (20) according to claim 1, **characterised in that** the specific reagent (30) further includes a buffer medium in order to maintain a sufficient ionic force.

3. Electrochemical sensor (20) according to claim 2, **characterised in that** the buffer medium is of the HEPES type in order to measure a higher signal.

4. Electrochemical sensor (20) according to any of the claims 1 to 3, **characterised in that** said at least one thromboplastin of the specific reagent (30) includes a composition in which the quantity of negative phospholipids is greater than the quantity of positive phospholipids.

5. Electrochemical sensor (20) according to claim 4, **characterised in that** the level of negative phospholipids, such as phosphatidylserin (PS) is greater than 65%.

6. Electrochemical sensor (20) according to any of the preceding claims, **characterised in that** the specific reagent (30) further includes in its composition an activator comprising a calcium salt such as CaCl₂.

7. Electrochemical sensor (20) according to any of the preceding claims, **characterised in that** said oligopeptidic derivative has, as terminal amino-acid, arginine (Arg) linked to one other amine function selected from among 2-aminobutyric acid (Abu), alanine (Ala), 3-cychlohexylalanine (Cha), 2-cyclohexylglycine (Chg), phenylalanine (Phe), pipecolic acid (Pip), proline (Pro), valine (Val) and glycine (Gly) said amino-acids being able to be in the L, D or DL form.

8. Electrochemical sensor (20) according to claim 7, **characterised in that** said charged portion (LG) is linked to said arginine (Arg) and comprises an amino-aryl or amino-heteroaryl group (LG) selected from among aniline, quinolylamine and naphtylamine derivatives possibly substituted by one or more substituents selected from among an halogen, an hydroxy, amino, nitro, alkyl, alkoxy, alkanoyl, anilino and aminophenyl radical, which may be substituted.

9. Electrochemical sensor (20) according to any of the preceding claims, **characterised in that** the substrate including an oligopeptidic derivative further includes to the opposite end of the charged portion (LG) a protective group (R₁) in order to immobilise said substrate onto the working electrode (24a).

10. Electrochemical sensor (20) according to claim 9, **characterised in that** the protective group (R₁) selected from among the Boc (ter-butoxycarbonyl), Tos (paratoluenesulfonic), t-Bups (ter-butylphenylsulfonyl), Mes (methylsulfonyl), Naps (2-naphtylsulfonyl), Bzo (benzoyl), Z (benzyloxycarbonyl), isopropylsulfonyl, camphosulfonyl acids.

11. Electrochemical sensor (20) according to any of the preceding claims, **characterised in that that** the oligopeptidic derivative includes a salt formed with hydrochloric acid (HCl), acetic acid or trifluoroacetic acid (TFA).

12. Electrochemical sensor (20) according to any of the preceding claims, **characterised in that** the substrate including an oligopeptidic derivative is selected from among:
- Z-Gly-Pro-Arg-3-chloro-4-hydroxyanilide
- Tos-Gly-Pro-Arg-3-chloro-4-hydroxyanilide
- Boc-(D)-Chg-Gly-Arg-3-chloro-4-hydroxyanilide
- H-(D)-Chg-Gly-Arg-3-chloro-4-hydroxyanilide
- Z-Gly-Pro-Arg-2- chloro-4-hydroxyanilide
and their compatible mineral or organic salts.

13. Electronic measuring apparatus (10) for determining prothrombin time (PT) of a drop of whole blood deposited on a measuring zone (29) of a sensor (20) according to claims 1 to 12 connected to said apparatus including:
- a case (11) housing a power source and allowing said sensor to be connected;
- an electronic circuit allowing a voltage or intensity to be modulated between the electrodes (24a, 25a) of said sensor, a signal representative of the prothrombin time (PT) to be interpreted, and displayed on a display panel (12).

14. Electronic measuring apparatus (10) according to claim 13, **characterised in that** the power source is an autonomous power source such as a battery or an accumulator.

15. Electronic measuring apparatus (10) according to claim 13 or 14, **characterised in that** it further includes temperature sensing means (15) in proximity to contact zone (14) of said sensor to adjust the electric signal parameters as a function of the ambient temperature.

16. Amperometric method for determining the prothrombin time (PT) of a drop of whole blood deposited in the measuring zone (29) of an electrochemical sensor (20) according to any of the claims 1 to 12 introduced into an electronic measuring apparatus (10) according to any of the claims 13 to 15, **characterised in that** the electronic circuit of said measuring apparatus allows:
- the presence of a drop of blood to be detected to establish a determined voltage across the electrodes (24a, 25a);
- a curve to be recorded giving the current variation as a function of time;
- the point of change of direction of said curve which corresponds to the maximum thrombin release speed to be determined by means of measuring windows;
- the co-ordinates of said point to be correlated with a prothrombin time (PT) value, for example by means of a calibration table; and
- said value to be displayed on a display panel (12).

## Patentansprüche

1. Elektrochemischer Sensor (20) für die Messung eines repräsentativen Wertes (PT), der die Koagulationszeit eines Tropfens Vollblut repräsentiert, **dadurch gekennzeichnet, dass** er umfasst:
- einen isolierenden Träger (21, 22) in Form einer Zunge, die wenigstens eine Referenzelektrode (25a) und eine Arbeitselektrode (24a) trägt und eine Messzone (29) aufweist, die dazu bestimmt ist, eine Vollblut-Probe aufzunehmen;
- wobei die Arbeitselektrode (24a) mit einem spezifischen Reagenz (30) in trockener Form beschichtet ist, dessen Zusammensetzung wenigstens ein Thromboplastin und ein Substrat enthält, das ein Oligopeptid-Derivat enthält, das eine stereospezifische Konfiguration eines Ortes des Thrombinenzyms besitzt, um die Abtrennung eines beladenen Abschlussteils (LG) dieses Substrats mittels dieses Letzteren zu ermöglichen.

2. Elektrochemischer Sensor (20) nach Anspruch 1, **dadurch gekennzeichnet, dass** das spezifische Reagenz (30) außerdem ein Puffermedium enthält, um eine ausreichende ionische Kraft aufrecht zu erhalten.

3. Elektrochemischer Sensor (20) nach Anspruch 2, **dadurch gekennzeichnet, dass** das Puffermedium vom HEPES-Typ ist, um ein höheres Signal zu erhalten.

4. Elektrochemischer Sensor (20) nach einem der Ansprüche 1 bis 3, **dadurch gekennzeichnet, dass** das wenigstens eine Thromboplastin des spezifischen Reagenzes (30) eine Zusammensetzung enthält, in der die Menge negativer Phospholipide größer als die Menge positiver Phospholipide ist.

5. Elektrochemischer Sensor (20) nach Anspruch 4, **dadurch gekennzeichnet, dass** der Anteil negativer Phospholipide wie etwa Phosphatidylserin (PS) größer als 65 % ist.

6. Elektrochemischer Sensor (20) nach einem der vorhergehenden Ansprüche, **dadurch gekennzeichnet, dass** das spezifische Reagenz (30) außerdem in seiner Zusammensetzung einen Aktivierer enthält, der ein Calciumsalz wie etwa CaCl₂ enthält.

7. Elektrochemischer Sensor (20) nach einem der vorhergehenden Ansprüche, **dadurch gekennzeichnet, dass** das Oligopeptid-Derivat als Aminosäurenabschluss Arginin (Arg) enthält, das mit wenigstens einem anderen Aminosäurenradikal verbunden ist, das gewählt ist aus 2-Aminobuthyrin-Säure (Abu), Alanin (Ala), 3-Cyclohexylalanin (Cha), 2-Cyclohexylglycin (Chg), Phenylalanin (Phe), Pipecolinsäure (Pip), Prolin (Pro), Valin (Val) und Glycin (Gly), wobei diese Aminosäuren in L-, D- oder DL-Form vorliegen können.

8. Elektrochemischer Sensor (20) nach Anspruch 7, **dadurch gekennzeichnet, dass** die Beladung (LG) mit Arginin (Arg) verbunden ist und eine Aminoaryl- oder Aminoheteroaryl-Gruppe (LG) enthält, die gewählt ist aus den Derivaten von Alinin, Quinolylamin und Naphthalamin, die eventuell durch einen oder mehrere Substituenten substituiert sind, die gewählt sind aus einem Halogen, einem Hydroxy-, Amino-, Nitro-, Alkyl-, Alkoxy-, Alkanoyl-, Anilino- und einem Aminophenyl-Radikal, das eventuell substituiert ist.

9. Elektrochemischer Sensor (20) nach einem der vorhergehenden Ansprüche, **dadurch gekennzeichnet, dass** das Substrat ein Oligopeptid-Derivat enthält, das an dem der Beladung (LG) gegenüberliegenden Ende eine Protektorgruppe (R₁) aufweist, um das Substrat auf der Arbeitselektrode (24a) zu immobi-lisieren.

10. Elektrochemischer Sensor (20) nach Anspruch 9, **dadurch gekennzeichnet, dass** die Protektorgruppe (R₁) gewählt ist aus den Säuren Boc (Ter-Butoxycarbonyl), Tos (Para-Toluensulfon), T-Bups (Ter-Butylphenylsulfonyl), Mes (Methylsulfonyl), Naps (2-Naphthylsulfonyl), Bzo (Benzoyl), Z (Benzyloxycarbonyl), Isopropylsulfonyl, Camphosulfonyl.

11. Elektrochemischer Sensor (20) nach einem der vorhergehenden Ansprüche, **dadurch gekennzeichnet, dass** das Oligopeptid-Derivat aus einem Salz besteht, das mit Hydrochlorsäure (HCL), mit Essigsäure oder mit Trifluoressigsäure (TFA) gebildet ist.

12. Elektrochemischer Sensor (20) nach einem der vorhergehenden Ansprüche, **dadurch gekennzeichnet, dass** das Substrat ein Oligopeptid-Derivat enthält, das gewählt ist aus:
- Z-Gly-Pro-Arg-3-Chlor-4-Hydroxyanilid
- Tos-Gly-Pro-Arg-3-Chlor-4-Hydroxyanilid
- Boc-(D)-Chg-Gly-Arg-3-Chlor-4-Hydroxyanilid
- H-(D)-Chg-Gly-Arg-3-Chlor-4-Hydroxyanilid
- Z-Gly-Pro-Arg-2-Chlor-4-Hydroxyanilid
und ihren kompatiblen mineralischen oder organischen Salzen.

13. Tragbare elektronische Messvorrichtung (10) für die Bestimmung der Prothrombin-Zeit (PT) eines Tropfens Vollblut, der auf eine Messzone (29) eines mit der Vorrichtung verbundenen Sensors (20) nach einem der Ansprüche 1 bis 12 aufgebracht ist, **dadurch gekennzeichnet, dass** sie umfasst:
- ein Gehäuse (11), das eine Energiequelle aufnimmt und ermöglicht, den Sensor anzuschließen, und
- eine elektronische Schaltung, die ermöglicht, eine Spannung oder eine Stärke zwischen den Elektroden (24a, 25a) des Sensors zu modulieren, ein die Prothrombin-Zeit (PT) repräsentierendes Signal zu interpretieren und den Wert auf einem Schirm (12) anzuzeigen.

14. Messvorrichtung (10) nach Anspruch 13, **dadurch gekennzeichnet, dass** die Energiequelle eine eigenständige Energiequelle wie etwa eine Batteriezelle oder ein Akkumulator ist.

15. Messvorrichtung (10) nach Anspruch 13 oder 14, **dadurch gekennzeichnet, dass** sie außerdem Temperaturmessmittel (15) in der Nähe der Anschlusszone (14) des Sensors aufweist, um die Parametrierung des elektrischen Signals in Abhängigkeit von der Umgebungstemperatur anzupassen.

16. Verfahren zum ampèrometrischen Bestimmen der Prothrombin-Zeit (PT) eines Tropfens Vollblut, der auf eine Messzone (29) eines in eine elektronische Messvorrichtung (10) nach einem der Ansprüche 13 bis 15 eingesetzten elektrochemischen Sensors (20) nach einem der Ansprüche 1 bis 12 aufgebracht ist, **dadurch gekennzeichnet, dass** die elektronische Schaltung der Messvorrichtung ermöglicht:
- das Vorhandensein eines Bluttropfens zu erfassen, um zwischen den Elektroden (24a, 25a) eine bestimmte Spannung aufzubauen;
- eine Kurve aufzuzeichnen, die die Veränderung des Stroms als Funktion der Zeit angibt;
- mittels Messfenstern den Wendepunkt der Kurve zu bestimmen, der der maximalen Freisetzungsgeschwindigkeit von Thrombin entspricht;
- beispielsweise mittels einer Eichtabelle die Koordinaten des Punkts mit einem Prothrombin-Zeitwert (PT) zu korrelieren und
- den Wert auf einem Anzeigeschirm (12) anzuzeigen.
